# EUROPEAN PATENT SPECIFICATION

(11) **EP 1 744 724 B1**
(45) Date of publication and mention of the grant of the patent: **25.08.2010**
(21) Application number: 04815773.9
(22) Date of filing: 29.12.2004
(51) Int. Cl.: A61K 8/04, A61K 8/34, A61K 8/44, A61Q 9/02

(54) **SELF-FOAMING SHAVE GEL PRODUCTS**
SELBSTSCHÄUMENDE RASIERGELPRODUKTE
PRODUITS DE GEL A RASER AUTO-MOUSSANTS

(30) Priority: 12.03.2004 US 552686 P
(43) Date of publication of application: 24.01.2007
(73) Proprietor: The Gillette Company, Boston, Massachusetts 02199 (US)
(72) Inventor: MCLAUGHLIN, Ronald, Reading, MA 01867 (US)
(74) Representative: Wilding, Richard Alan
(86) International application number: PCT/US2004/043771
(87) International publication number: WO 2005/094764

(56) References cited:
- EP-A- 0 194 097
- WO-A-02/41860
- WO-A-02/087520
- GB-A- 2 325 936
- US-A- 5 500 211
- US-A- 5 902 574
- US-B1- 6 352 689

## Description

This invention relates to non-soap shave gel compositions, such as non- soap self-foaming shave gel compositions.

Post-foaming or self-foaming shave gels are now well-known and have been described, for example, in U. S. Pat. Nos. 2,995, 521 (Bluard), 3,541, 581 (Monson), 4,405, 489 (Sisbarro), 4,528, 111 (Su), 4,651, 503 (Anderson), 5, 248, 495 (Patterson), 5,308, 643 (Osipow), 6,352,689 (Szymczak) and 5,326, 556(Bamet) and published PCT application WO 91/07943 (Chaudhuri). Such compositions generally take the form of an oil-in-water emulsion in which the self-foaming agent, generally a volatile (i. e. low boiling point) aliphatic hydrocarbon, is solubilized in the oil phase, and the water phase comprises a water-soluble soap component. The product is generally packaged in an aerosol container with a barrier, such as a piston or collapsible bag, to separate the self-foaming gel from the propellant required for expulsion of the product. The product is dispensed as a clear, translucent or opaque gel that is substantially free from foaming until it is spread over the skin, at which time it produces a foam lather generated by the volatilization of the volatile hydrocarbon foaming agent.

While the conventional self-foaming shave gels have gained wide acceptance by consumers, they can be somewhat harsh and drying to the skin due to the soap component. To counteract this effect, the typical shave gel composition is formulated with skin soothing components such as humectants, emollients, silicones, etc.

While the addition of such components substantially improves the aesthetics of the product, repeated use can still produce undesirable drying of the skin, particularly among female users. Accordingly, soap-free, self-foaming shave gel compositions are disclosed in U. S. 5,500, 211 and US 2004/0018167.

The present invention relates to a soap-free self-foaming shave gel composition which has the superior performance attributes typically associated with a soap-based shave gel, such as relatively high lather density, creaminess, spread consistency, good coverage, and pleasant feel, but does not have the harsh and drying attributes of some soap-based shave preparations. The shave gel composition of the present invention can include, for example, water, a short-chain polyol, a water-soluble N-acyl sarcosinate salt, and a volatile self-foaming agent.

The invention features a shaving composition according to claim 1.

### DESCRIPTION OF DRAWINGS

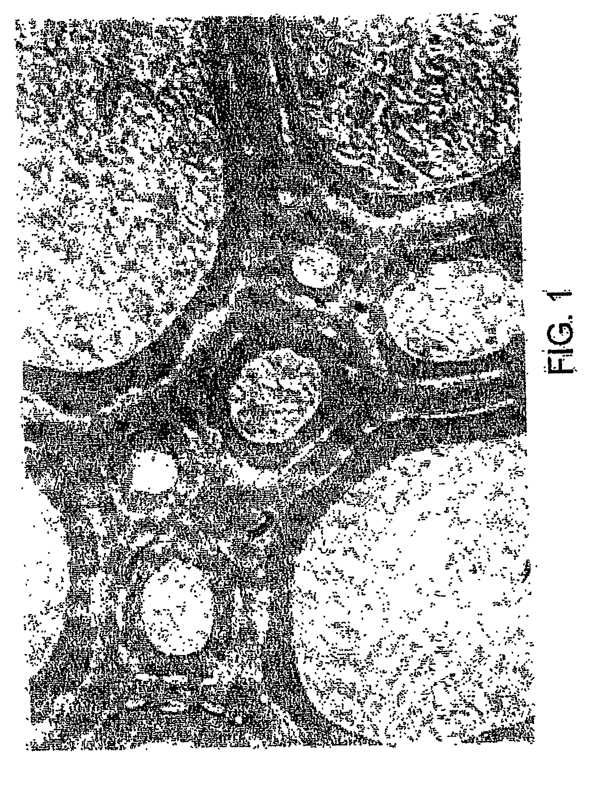

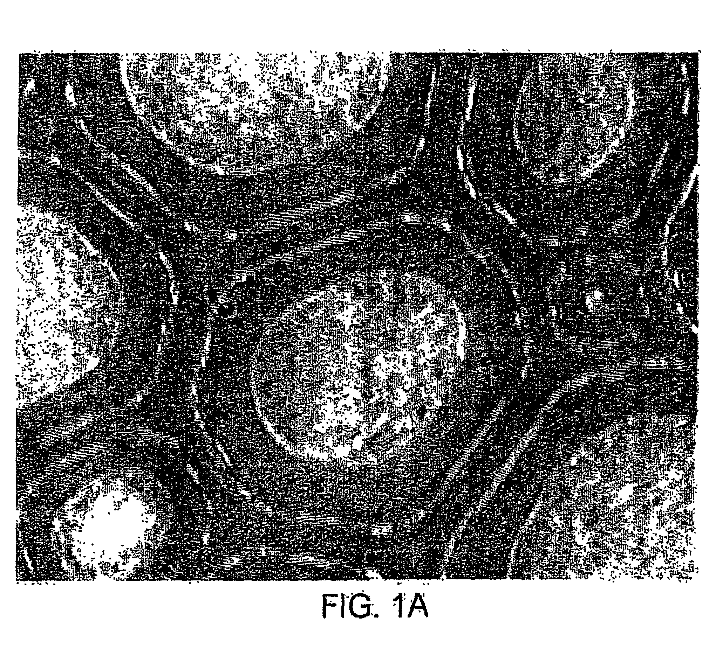

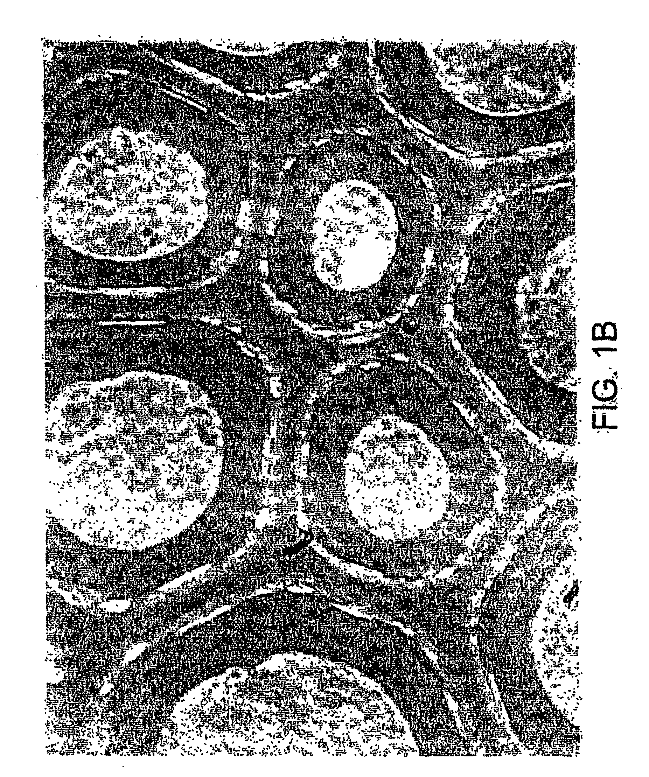

FIGS. 1-1B are photomicrographs of lathers of shaving compositions. The shaving composition of the present invention includes water, a short-chain polyol, comprising Propylene glycol N-acyl sarcosine wherein the acyl group has 10 to 20 carbon atoms, sufficient base to solubilize the N-acyl sarcosine and provide a pH of from about 4 to about 8, and a self-foaming agent. The composition is in the form of a self-foaming gel and is substantially free of soap. A more preferred shaving composition further includes a non-ionic surfactant (e.g., Laureth-4 and/or Laureth-23), a fatty alcohol, and a gelling aid, and is substantially free of anionic surfactants that are not N-acyl sarcosinates.

The short-chain polyol is a polyhydric alcohol having from 3 to 6 carbon atoms and from 2 to 6 hydroxyl groups. Examples of short-chain polyols include glycerin, propylene glycol, butylene glycol, and diglycerin. The shaving composition can include up to about 5.0% of the short-chain polyol. If more than about 5.0% of the short-chain polyol is used, the shaving composition may tend to become too soft and/or the emulsion can be broken; if very low levels are used, e.g., less than 0.15%, the desired lathering properties may not be obtained. The shaving composition includes from about 0.25% to about 5.0%, more preferably from about 0.5% to about 2%, of a short-chain polyol In some embodiments, the shaving composition can include a mixture of two or more short-chain polyols. In embodiments in which the shaving composition includes a mixture of short-chain polyols, the sum of the weight percents of each short-chain polyol in the cornposition is within the weight percent ranges listed above. The shaving composition includes propylene glycol, or preferably a mixture of glycerin and propylene glycol.

The short-chain polyols can substantially improve the lathering characteristics and/or the stability of the shaving composition. The lathers of shaving compositions that include a short-chain polyol tend to exhibit a more uniform, consistent bubble structure (the bubbles are formed by the dispersion of propellant through the shaving composition) and a thicker, more defined, more uniform interstitial region than similar compositions that do not include the short-chain polyol. Without being bound by theory, it is believed that the short-chain polyols reduce the surface tension of the bubbles, resulting in a more stable lather, and work synergistically with the surfactant(s) to reduce the size of the bubbles, resulting in an increased bubble surface area. The smaller bubble size and the more consistent bubble structure can allow for tighter bubble packing, which can make the lather creamier and more stable, and can make the foam more consistent. The smaller bubble size may also make the foam stiffer.

FIG. 1 shows a lather of a shaving composition that does not include any short-chain polyols, while FIG. 1A shows a lather of a shaving composition with the same formulation as the shaving composition shown in FIG. 1, except for the further addition of glycerin. FIG. 1B shows a lather of a shaving composition with the same formulation as the shaving composition shown in FIG. 1, except for the further addition of propylene glycol. The generally circular regions shown in FIGS. 1-1B are the bubbles, and the space between the bubbles is the interstitial region. The lather of the shaving composition that does not include any short-chain polyols (FIG. 1) has a less uniform bubble structure and a less defined interstitial region than the lathers of the shaving compositions that include a short-chain polyol (FIGS. 1A and 1B). The interstitial regions in the lathers of the shaving compositions with the short-chain polyols also are thicker than the interstitial region in the lather of the shaving composition without any short-chain polyols. The short-chain polyol can reduce interstitial drainage by increasing water viscosity; reduced interstitial drainage can improve lather stability and richness. The well-defined and more uniform bubble structure of the lathers of the shaving compositions that include a short-chain polyol can enhance those lathers (e.g., can cause the lathers to have relatively high lather density, creaminess, spread consistency, good coverage, pleasant feel) and generally makes the lathers more resistant to breaking down.

The N-acyl sarcosine may be selected from any of those with an acyl moiety with from 10 to 20, preferably from 12 to 18, carbon atoms, that will provide a water-soluble sarcosinate when neutralized with an appropriate base. These typically include stearoyl sarcosine, myristoyl sarcosine, oleoyl sarcosine, lauroyl sarcosine, cocoyl sarcosine and mixtures thereof (e.g., Hamposyl SM). Stearoyl sarcosine and myristoyl sarcosine, as well as mixtures thereof (e.g., a 3:1 stearoyl sarcosine:myristoyl sarcosine mixture), are preferred. In some embodiments, the shaving composition includes from about 4% to about 16%, preferably from about 6% to about 12%, of the N-acyl sarcosine. It is also possible to utilize a pre-neutralized sarcosinate, such as triethanolamine myristoyl sarcosinate, in which case it will not be necessary to separately add base to the composition except for such amount of acid or base as may be required to adjust the pH of the final composition. Both the sarcosine component and the base component preferably are selected so as to provide a clear or translucent gel when combined with the other components of the composition.

The base can be selected from any of the organic amine bases which are typically utilized to neutralize N-acyl sarcosines to form water-soluble salts. These include, for example, isopropanolamine, mono-, di- and triethanolamine, aminomethyl propanol and aminomethyl propanediol. Triethanolamine is preferred. The amount of base which is utilized will depend on the amount of sarcosine which is present in the composition. A sufficient amount of base should be utilized to solubilize the sarcosine in the aqueous phase of the composition and provide a pH of from about 4 to about 8, preferably from about 5 to about 7. To arrive at this pH range the sarcosine must be from about 50% to about 90% neutralized, preferably from about 60% to about 80% neutralized. It is, therefore, most preferred that there be at least a slight molar excess of sarcosine to base. Typically, the base will comprise from about 1% to about 6% of the composition.

The self-foaming agent can be any volatile hydrocarbon or halogenated hydrocarbon with a boiling point that is low enough to cause the hydrocarbon to volatilize and foam the gel upon application to the skin, but high enough to prevent the hydrocarbon from causing the gel to foam prematurely. The typical boiling point of such an agent generally falls within the range of from about -20°C to about 40°C. The self-foaming agent will normally be selected so as to provide a vapor pressure at 20°C of about 3 to about 20 psig, preferably about 5 to about 15 psig. Preferred self-foaming agents are selected from saturated aliphatic hydrocarbons having from 4 to 6 carbon atoms, such as n-pentane, isopentane, neopentane, n-butane, isobutane, and mixtures thereof Most preferred is a mixture of isopentane and isobutane in a weight ratio of about 1:1 to about 3:1 (e.g., about 3:1 isopentane:isobutane). The self-foaming agent will normally be present in an amount comprising from about 1% to about 8% of the composition, preferably from about 2% to about 5%.

The shaving composition can also include a gelling agent and, for example, the composition may include up to about 10%, and preferably up to about 7%, of a non-volatile paraffinic hydrocarbon fluid which aids in gelling the composition. The terms "non-volatile" and "fluid" mean that these materials are liquid at room temperature and have a boiling point above 200°C. Such hydrocarbon fluids include mineral oils, petrolatum (which can, e.g., make a gel stiffer and/or can help solubilize propellant), and branched-chain aliphatic liquids. These fluids typically have from about 16 to about 48 carbon atoms, preferably from about 20 to about 40 carbon atoms, and a kinematic viscosity (measured using method ASTM D445) of from about 5 centistokes (cSt) to about 100 centistokes, preferably from about 10 centistokes to about 70 centistokes, at 40°C. Preferred non-volatile paraffinic hydrocarbon fluids include mineral oil with a kinematic viscosity of from about 10 centistokes to about 70 centistokes at 40°C, hydrogenated polyisobutene with a molecular weight of from about 320 to about 420, and mixtures thereof.

It may also be desirable to include a water-soluble gelling aid or thickening agent in the shaving composition to improve the consistency and stability of the gel, as well as to adjust its viscosity. These may include, for example, hydroxyalkyl cellulose polymers such as hydroxyethyl cellulose and hydroxypropyl cellulose (sold under the trademarks "Natrosol" and "Klucel" respectively), copolymers of acrylic acid and polyallyl sucrose (sold under the trademark "Carbopol"), carboxymethyl cellulose, and cellulose methyl ether (sold under the trademark "Methocel"). Natural or synthetic gums, resins, and starches may also be used. The preferred thickening agents are hydroxyethyl cellulose, hydroxypropyl cellulose, and mixtures thereof. The gelling aid or thickening agent is typically included in an amount of from about 0.01 % to about 5% (e.g., from about 0.05% to about 2%), preferably from about 0.01% to about 2%.

Water is the major component of the composition and is used in sufficient quantities to solubilize the surfactant component and form the continuous phase of the emulsion, while providing a stable gel of suitable viscosity with desirable lathering and rinsing properties. The water is added to the shaving composition in a sufficient amount

(q.s.) to bring the total weight percent of all components to 100%. The quantity of water in the composition typically falls within the range of from about 65% to about 85%, preferably from about 70% to about 80%.

In addition to the above-described components, the shaving composition of the present invention can include a variety of other well-known cosmetic ingredients to improve the aesthetics and performance characteristics of the composition.

It is generally desirable to include up to about 10%, preferably up to about 9%, more preferably about 4% to about 8%, e.g. 7.7%, of a non-ionic surfactant in the composition to improve foam quality, wettability, gel consistency, and rinsability. Suitable non-ionic surfactants will typically have a Hydrophilic-Lipophilic Balance (HLB) of 9 or more and will be compatible with the aqueous sarcosinate component. Preferred non-ionic surfactants include the polyoxyethylene ethers of fatty alcohols, acids and amides, particularly those having from 10 to 20 carbon atoms, preferably from 12 to 18 carbon atoms, in the fatty moiety, and from about 2 to about 60, preferably from 4 to 30, ethylene oxide units. These include, for example, Oleth-20, Steareth-21, Ceteth-20, Laureth-4, and Laureth-23. In a preferred embodiment, the shaving composition includes a mixture of Laureth-4 and Laureth-23. Other examples of non-ionic surfactants include the polyoxyethylene ethers of alkyl substituted phenols, such as Nonoxynol-4 and Nonoxynol-20, fatty alkanolamides such as Lauramide DEA and Cocamide MEA, polyethoxylated sorbitan esters of fatty acids, such as Polysorbate-20, lauryl polyglucoside, sucrose laurate, and polyglycerol 8-oleate.

The shaving composition also preferably includes up to about 8%, preferably from about 2% to about 6%, of a fatty alcohol such as myristyl, lauryl and stearyl alcohol and octyl dodecanol. The term "fatty" means that the fatty alcohol includes from 10 to 20 carbon atoms, and preferably from 12 to 18 carbon atoms.

It is particularly desirable to include in the composition a cationic conditioning polymer which is substantive to the skin in order to improve lubricity and post-shave skin feel. Such polymers may include polymeric quaternary ammonium salts of hydroxyethyl cellulose such as polyquatemium-10 and polyquaternium-24. These polymers are typically included in the shaving composition in an amount of from about 0.05% to about 2%, preferably from about 0.1% to about 1%.

Other useful additives which may be utilized in the composition include humectants such as sorbitol, emollients including fatty esters such as isopropyl myristate, decyl oleate, 2-ethylhexyl palmitate, PEG-7 glyceryl cocoate, and glyceryl linoleate, propoxylated fatty ethers such as PPG-10 cetyl ether and PPG-11 stearyl ether, di- and triglycerides such as lecithin and caprylic/capric triglyceride, vegetable oils, PEG-10 soy sterol, and similar materials, skin freshening and soothing agents such as menthol, aloe (e.g., aloe barbadensis leaf juice), allantoin, lanolin, bisabolol, leaf oil (e.g., eucalyptus globulus leaf oil, rosmarinum officinalis leaf oil, melaleuca alternifolia leaf oil, mentha viridis leaf oil, collagen and hyaluronic acid, lubricants such as polyethylene oxide (e.g., PEG-14M, PEG-23M), fluorosurfactants, and silicones (e.g., dimethicone, dimethiconol, dimethicone copolyol, stearyl dimethicone, cetyl dimethicone copolyol, phenyl dimethicone, cyclomethicone, etc.), vitamins (including vitamin precursors and derivatives) such as panthenol, tocopheryl acetate, niacinamide, retinyl palmitate, and vitamin A palmitate, colorants (e.g., FD&C Blue #1), fragrances, antioxidants, antibacterial and/or antifungal agents (e.g., triclosan), and preservatives (e.g., methylchloroisothiazolinone, methylisothiazolinone, DMDM hydantoin, iodopropynyl butycarbamate).

A preferred shaving composition of the present invention includes, in percent by weight, from about 65% to about 85% water, about 0.25% to about 5.0% of a short-chain polyol according to claim 1 from about 4% to about 16% N-acyl sarcosine (wherein the acyl group has from 10 to 20, preferably from 12 to 18, carbon atoms), sufficient organic amine base to solubilize the N-acyl sarcosine and provide a pH of from about 4 to about 8, from about 1% to about 8% self-foaming agent, up to about 8% of a non-ionic surfactant, and from about 1% to about 8% of a fatty alcohol Most preferably the composition according to claim 1 includes from about 70% to about 80% water, from about 6% to about 12% N-acyl sarcosine, sufficient base to provide a pH of from about 5 to about 7, from about 2% to about 5% self-foaming agent, from about 1% to about 10% of a non-ionic surfactant, from about 2% to about 6% of a fatty alcohol, and from about 0.01% to about 2% of a thickening agent.

The shaving composition of the present invention may be packaged in any dispenser suitable for dispensing post-foaming shave gels. These include aerosol containers with a barrier, such as a collapsible bag or piston, to separate the gel from the propellant required for expulsion, collapsible tubes, and pump or squeeze containers.

The allowing examples are intended to be illustrative and non-limiting. All parts and percentages are by weight.

| | EXAMPLE 1 | EXAMPLE (Comparative) 2 | EXAMPLE (Comparative) 3 | EXAMPLE 4 |
|---|---|---|---|---|
| Purified Water | 73.3351 | 74.7853 | 72.8623 | 72.8623 |
| Stearoyl Sarcosine & | 6.7305 | 6.7305 | 6.7305 | 6.7305 |
| Myristoyl Sarcosine (3:1) | | | | |
| Laureth-23 | 6.0575 | 5.7690 | 5.7690 | 5.7690 |
| Isobutane & Isopentane (3:1) | 3.8500 | 3.8500 | 3.8500 | 3.8500 |
| Myristyl Alcohol | 2.7884 | 2.6922 | 2.6922 | 2.6922 |
| Triethanolamine (99%) | 2.4518 | 2.4518 | 2.4518 | 2.4518 |
| Laureth-4 | 1.7307 | 1.7307 | 1.7307 | 1.7307 |
| Glycerin | 0.9615 | | 1.9230 | |
| Propylene Glycol | 0.0192 | | | 1.9230 |
| Mineral Oil | 0.8654 | | | |
| Petrolatum | | 0.4808 | 0.4808 | 0.4808 |
| Polyquaternium-10 | 0.2404 | 0.2404 | 0.2404 | 0.2404 |
| PEG-10 Soy Sterol | 0.2404 | 0.2404 | 0.2404 | 0.2404 |
| PEG-14M | | 0.2404 | 0.2404 | 0.2404 |
| PEG-23M | 0.2404 | | | |
| Hydroxyethylcellulose | 0.0962 | 0.0962 | 0.0962 | 0.0962 |
| Tocopheryl Acetate | 0.0962 | | | |
| Niacinamide | 0.0962 | | | |
| DMDM Hydantoin & Iodo- | 0.0577 | 0.0577 | 0.0577 | 0.0577 |
| propynyl Butylcarbarnate (19:1) | | | | |
| Eucalyptus Globulus Leaf Oil & Rosmarinum Officinalis Leaf Oil & Melaleuca Alternifolia Leaf Oil & Mentha Viridis Leaf Oil (60:35:4: 1) | 0.0481 | | | |
| Methylduoroisothiazolmone & Methylisothiazolinone (3.3:1) | 0.0385 | 0.0385 | 0.0385 | 0.0385 |
| Hydroxypropylcelluose | 0.0192 | 0.0192 | 0.0192 | 0.0192 |
| Aloe Barbadensis Leaf Juice | 0.0096 | | | |
| Triclosan | 0.0096 | | | |
| Retinyl Palmitate | 0.0096 | | | |
| Bisabolol | 0.0077 | | | |
| Fragrance | | 0.5769 | 0.5769 | 0.5769 |
| FD&C Blue #1 | 0.0001 | | | |
| TOTAL | 100 | 100 | 100 | 100 |

FIGS. 1-1B show photomicrographs of the shaving compositions of Examples 2-4, respectively, and are described in further detail above.

The above example compositions were prepared according to the following procedure.

### Procedure

The glycerin and/or propylene glycol, hydroxyethyl cellulose, polyquatemium-10, and PEG-14-M or PEG-23M were dissolved into the water at room temperature with stirring.

After about 40 minutes of stirring, the aqueous solution was heated to about 85°C, and the sarcosine (which had been pre-melted), myristyl alcohol, Laureth-23, PEG-10 Soy Sterol and Laureth-4 were added. The solution and the components were mixed for about 10 minutes.

The triethanolamine was added, and the mixing was continued at about 85°C for about 30 minutes.

The mixture was cooled to 70°C, and the mineral oil and/or petrolatum and triclosan were added. The mixture was then mixed for 10 minutes.

The mixture was cooled to 50°C, and the preservatives were added. The mixture was mixed for 10 minutes.

The mixture was cooled to 30°C, and the fragrance, colorant, aloe gel, retinyl palmitate, bisabolol, tocopheryl acetate, leaf oils and hydroxypropyl cellulose were added The hydroxypropyl cellulose was first premixed with about 0.5 parts of water at 55°C, and then with an additional 3.5 parts of water at room temperature.

After cooling to room temperature the mixture was blended with the isopentane/isobutene to form the final composition.

As used in this application, all percentages are by weight on a solids basis, unless indicated otherwise. Additionally, the weight percents provided for a given component preferably apply to mixtures of that component, as well.

## Claims

1. A shaving composition in the form of a self-foaming gel comprising, in percent by weight, about 65% to about 85% water, about 0.25% to about 5.0% of a short-chain polyol having from 3 to 6 carbon atoms and from 2 to 6 hydroxyl groups, the short-chain polyol comprising propylene glycol, about 4% to about 16% N-acyl sarcosine wherein the acyl group has from 10 to 20 carbon atoms sufficient organic amine base to solubilize the N-acyl sarcosine and provide a pH of from about 4 to about 8, and about 1 % to 8% self-foaming agent, said composition being substantially free of soap.

2. The shaving composition of claim 1, wherein the N-acyl sarcosine is selected from the group consisting of stearoyl sarcosine, myristoyl sarcosine, oleoyl sarcosine, lauroyl sarcosine, cocoyl sarcosine and mixtures thereof.

3. The shaving composition of claim 2, wherein the short-chain polyol is selected from the group consisting of glycerin, propylene glycol, butylene glycol, diglycerin, and combinations thereof.

4. The shaving composition of claim 3, wherein the organic amine base is triethanolamine.

5. The shaving composition of claim 4, wherein the short-chain polyol comprises glycerin.

6. The shaving composition of claim 4, wherein the self-foaming agent is a volatile hydrocarbon having from 4 to 6 carbon atoms or a mixture of such hydrocarbons.

7. The shaving composition of claim 6, further comprising up to about 10% of a non-ionic surfactant.

8. The shaving composition of claim 7, further comprising up to about 8% of a fatty alcohol.

9. The shaving composition of any of the preceding claims, wherein the shaving composition comprises about 0.5% to about 2% of the short-chain polyol.

10. The shaving composition of any of the preceding claims, wherein the shaving composition comprises about 70% to about 80% water, 0.5% to about 2% of the short-chain polyol, about 6% to about 12% N-acyl sarcosine, sufficient organic amine base to provide a pH of from about 5 to about 7, about 4% to about 8% non-ionic surfactant, and about 2% to about 5% self-foaming agent, said composition being substantially free of anionic surfactants that are not N-acyl sarcosinates.

11. The shaving composition of claim 10, further comprising about 0.05% to about 2% of a cationic conditioning polymer.

12. The shaving composition of claim 11, further comprising from about 0.01 % to about 5% of a thickening agent.

13. The shaving composition of claim 11, wherein the cationic conditioning polymer is a polymeric quaternary ammonium salt of hydroxyethyl cellulose.

14. The shaving composition of claim 12, wherein the thickening agent is selected from the group consisting of hydroxyethyl cellulose, hydroxypropyl cellulose, and mixtures thereof.

15. The shaving composition of any of the preceding claims, wherein the acyl group of the N-acyl sarcosine has from 12 to 18 carbon atoms.

16. The shaving composition of claim 10, wherein the N-acyl sarcosine is selected from the group consisting of stearoyl sarcosine, myristoyl sarcosine, oleoyl sarcosine, lauroyl sarcosine, cocoyl sarcosine and mixtures thereof, the organic amine base is triethanolamine, the short-chain polyol is selected from the group consisting of glycerin, propylene glycol, butylene glycol, diglycerin, and combinations thereof, and the self-foaming agent is a volatile hydrocarbon having from 4 to 6 carbon atoms or a mixture of such hydrocarbons.

17. The shaving composition of claim 8, wherein the self-foaming agent is a mixture of isopentane and isobutane in a weight ratio of from about 1: 1 to about 3: 1.

18. The shaving composition of claim 16, wherein the self-foaming agent is a mixture of isopentane and isobutane in a weight ratio of from about 1: 1 to about 3: 1.

## Patentansprüche

1. Rasierzusammensetzung in Form eines selbstschäumenden Gels, die, in Gewichtsprozent, etwa 65% bis etwa 85% Wasser, etwa 0,25% bis etwa 5,0% eines kurzkettigen Polyols mit 3 bis 6 Kohlenstoffatomen und 2 bis 6 Hydroxylgruppen, wobei das kurzkettige Polyol Propylenglycol umfasst, etwa 4% bis etwa 16% N-Acylsarcosin, in dem die Acylgruppe 10 bis 20 Kohlenstoffatome aufweist, ausreichend organische Aminbase, um das N-Acylsarcosin zu solubilisieren und einen pH von etwa 4 bis etwa 8 zu ergeben, und etwa 1 % bis 8% Selbstschäumungsmittel umfasst, wobei die Zusammensetzung im Wesentlichen seifenfrei ist.

2. Rasierzusammensetzung nach Anspruch 1, wobei das N-Acylsarcosin ausgewählt ist aus der Gruppe bestehend aus Stearoylsarcosin, Myristoylsarcosin, Oleoylsarcosin, Lauroylsarcosin, Cocoylsarcosin und Mischungen davon.

3. Rasierzusammensetzung nach Anspruch 2, wobei das kurzkettige Polyol ausgewählt ist aus der Gruppe bestehend aus Glycerin, Propylenglycol, Butylenglycol, Diglycerin und Kombinationen davon.

4. Rasierzusammensetzung nach Anspruch 3, wobei die organische Aminbase Triethanolamin ist.

5. Rasierzusammensetzung nach Anspruch 4, wobei das kurzkettige Polyol Glycerin umfasst.

6. Rasierzusammensetzung nach Anspruch 4, wobei das Selbstschäumungsmittel ein flüchtiger Kohlenwasserstoff mit 4 bis 6 Kohlenstoffatomen oder eine Mischung aus solchen Kohlenwasserstoffen ist.

7. Rasierzusammensetzung nach Anspruch 6, ferner bis zu etwa 10% ein nicht-ionisches Tensid umfassend.

8. Rasierzusammensetzung nach Anspruch 7, ferner bis zu etwa 8% Fettalkohol umfassend.

9. Rasierzusammensetzung nach einem der vorangehenden Ansprüche, wobei die Rasierzusammensetzung etwa 0,5% bis etwa 2% kurzkettiges Polyol umfasst.

10. Rasierzusammensetzung nach einem der vorangehenden Ansprüche, wobei die Rasierzusammensetzung etwa 70% bis etwa 80% Wasser, 0,5% bis etwa 2% kurzkettiges Polyol, etwa 6% bis etwa 12% N-Acylsarcosin, ausreichend organische Aminbase, um einen pH von etwa 5 bis etwa 7 zu ergeben, etwa 4% bis etwa 8% nicht-ionisches Tensid und etwa 2% bis etwa 5% Selbstschäumungsmittel umfasst, wobei die Zusammensetzung im Wesentlichen frei ist von anionischen Tensiden, bei denen es sich nicht um N-Acylsarcosinate handelt.

11. Rasierzusammensetzung nach Anspruch 10, ferner etwa 0,05% bis etwa 2% kationisches Konditionierungspolymer umfassend.

12. Rasierzusammensetzung nach Anspruch 11, ferner etwa 0,01% bis etwa 5% Verdickungsmittel umfassend.

13. Rasierzusammensetzung nach Anspruch 11, wobei das kationische Konditionierungspolymer ein polymeres quartäres Ammoniumsalz von Hydroxyethylcellulose ist.

14. Rasierzusammensetzung nach Anspruch 12, wobei das Verdickungsmittel ausgewählt ist aus der Gruppe bestehend aus Hydroxyethylcellulose, Hydroxypropylcellulose und Mischungen davon.

15. Rasierzusammensetzung nach einem der vorangehenden Ansprüche, wobei die Acylgruppe des N-Acylsarcosins 12 bis 18 Kohlenstoffatome aufweist.

16. Rasierzusammensetzung nach Anspruch 10, wobei das N-Acylsarcosin ausgewählt ist aus der Gruppe bestehend aus Stearoylsarcosin, Myristoylsarcosin, Oleoylsarcosin, Lauroylsarcosin, Cocoylsarcosin und Mischungen davon, die organische Aminbase Triethanolamin ist, das kurzkettige Polyol ausgewählt ist aus der Gruppe bestehend aus Glycerin, Propylenglycol, Butylenglycol, Diglycerin und Kombinationen davon, und das Selbstschäumungsmittel ein flüchtiger Kohlenwasserstoff mit 4 bis 6 Kohlenstoffatomen oder eine Mischung solcher Kohlenwasserstoffe ist.

17. Rasierzusammensetzung nach Anspruch 8, wobei das Selbstschäumungsmittel eine Mischung aus Isopentan und Isobutan in einem Gewichtsverhältnis von etwa 1:1 1 bis etwa 3:1 ist.

18. Rasierzusammensetzung nach Anspruch 16, wobei das Selbstschäumungsmittel eine Mischung aus Isopentan und Isobutan in einem Gewichtsverhältnis von etwa 1:1 1 bis etwa 3:1 ist.

## Revendications

1. Composition de rasage sous la forme d'un gel auto-moussant comprenant, en pour cent en poids, environ 65 % à environ 85 % d'eau, environ 0,25 % à environ 5,0 % d'un polyol à chaîne courte ayant de 3 à 6 atomes de carbone et de 2 à 6 groupes hydroxyles, le polyol à chaîne courte comprenant du propylène glycol, environ 4 % à environ 16 % de N-acyl sarcosine dans laquelle le groupe acyle a de 10 à 20 atomes de carbone, une quantité suffisante de base amine organique pour solubiliser la N-acyl sarcosine et fournir un pH allant d'environ 4 à environ 8, et d'environ 1 % à 8 % d'agent auto-moussant, ladite composition étant essentiellement dépourvue de savon.

2. Composition de rasage selon la revendication 1, dans laquelle la N-acyl sarcosine est choisie dans le groupe constitué de stéaroyl-sarcosine, myristoyl-sarcosine, oléoyl-sarcosine, lauroyl-sarcosine, cocoyl-sarcosine et leurs mélanges.

3. Composition de rasage selon la revendication 2, dans laquelle le polyol à chaîne courte est choisi dans le groupe constitué de glycérine, propylène glycol, butylène glycol, diglycérine, et leurs combinaisons.

4. Composition de rasage selon la revendication 3, dans laquelle la base amine organique est la triéthanolamine.

5. Composition de rasage selon la revendication 4, dans laquelle le polyol à chaîne courte comprend de la glycérine.

6. Composition de rasage selon la revendication 4, dans laquelle l'agent auto-moussant est un hydrocarbure volatil ayant de 4 à 6 atomes de carbone ou un mélange de tels hydrocarbures.

7. Composition de rasage selon la revendication 6, comprenant, en outre, jusqu'à environ 10 % d'un agent tensioactif non ionique.

8. Composition de rasage selon la revendication 7, comprenant, en outre, jusqu'à environ 8 % d'un alcool gras.

9. Composition de rasage selon l'une quelconque des revendications précédentes, où la composition de rasage comprend environ 0,5 % à environ 2 % du polyol à chaîne courte.

10. Composition de rasage selon l'une quelconque des revendications précédentes, où la composition de rasage comprend environ 70 % à environ 80 % d'eau, 0,5 % à environ 2 % du polyol à chaîne courte, environ 6 % à environ 12 % de N-acyl sarcosine, une quantité suffisante de base amine organique pour fournir un pH allant d'environ 5 à environ 7, environ 4 % à environ 8 % d'agent tensioactif non ionique, et environ 2 % à environ 5 % d'agent auto-moussant, ladite composition étant essentiellement dépourvue d'agents tensioactifs anioniques qui ne sont pas des N-acyl sarcosinates.

11. Composition de rasage selon la revendication 10, comprenant, en outre, d'environ 0,05 % à environ 2 % d'un polymère de conditionnement cationique.

12. Composition de rasage selon la revendication 11, comprenant, en outre, d'environ 0,01 % à environ 5 % d'un agent épaississant.

13. Composition de rasage selon la revendication 11, dans laquelle le polymère de conditionnement cationique est un sel d'ammonium quaternaire polymère d'hydroxyéthylcellulose.

14. Composition de rasage selon la revendication 12, dans laquelle l'agent épaississant est choisi dans le groupe constitué d'hydroxyéthylcellulose, hydroxypropylcellulose, et leurs mélanges.

15. Composition de rasage selon l'une quelconque des revendications précédentes, dans laquelle le groupe acyle de la N-acyl sarcosine a de 12 à 18 atomes de carbone.

16. Composition de rasage selon la revendication 10, dans laquelle la N-acyl sarcosine est choisie dans le groupe constitué de stéaroyl-sarcosine, myristoyl-sarcosine, oléoyl-sarcosine, lauroyl-sarcosine, cocoyl-sarcosine et leurs mélanges, la base amine organique est la triéthanolamine, le polyol à chaîne courte est choisi dans le groupe constitué de glycérine, propylène glycol, butylène glycol, diglycérine, et leurs combinaisons, et l'agent auto-moussant est un hydrocarbure volatil ayant de 4 à 6 atomes de carbone ou un mélange de tels hydrocarbures.

17. Composition de rasage selon la revendication 8, dans laquelle l'agent auto-moussant est un mélange d'isopentane et d'isobutane dans un rapport pondéral allant d'environ 1:1 à environ 3:1.

18. Composition de rasage selon la revendication 16, dans laquelle l'agent auto-moussant est un mélange d'isopentane et d'isobutane dans un rapport pondéral allant d'environ 1:1 à environ 3:1.
